# EUROPEAN PATENT SPECIFICATION

(11) **EP 4 042 973 B1**
(45) Date of publication and mention of the grant of the patent: **11.02.2026**
(21) Application number: 20912827.1
(22) Date of filing: 16.10.2020
(51) Int. Cl.: A61F 2/24

(54) **STRUCTURALLY FITTED TRANSCATHETER AORTIC VALVE IMPLANTATION DEVICE**
STRUKTURELL ANGEPASSTE TRANSKATHETER-AORTENKLAPPEN-IMPLANTATIONSVORRICHTUNG
DISPOSITIF D'IMPLANTATION DE VALVE AORTIQUE TRANS-CATHÉTER AJUSTÉE STRUCTURELLEMENT

(30) Priority: 09.01.2020 CN 202010021982
(43) Date of publication of application: 17.08.2022
(73) Proprietor: Nanjing Saint Medical Technology Co., Ltd., Nanjing, Jiangsu 210061 (CN)
(72) Inventor: MA, Chenming, Nanjing, Jiangsu 210032 (CN)
(74) Representative: Gille Hrabal Partnerschaftsgesellschaft mbB Patentanwälte
(86) International application number: PCT/CN2020/121635
(87) International publication number: WO 2021/139301

(56) References cited:
- WO-A1-2015/126711
- WO-A1-2016/055262
- WO-A1-2016/055262
- CN-A- 103 892 940
- CN-A- 107 405 197
- CN-A- 110 393 608
- CN-A- 111 110 402
- CN-U- 207 821 949
- CN-U- 208 864 574
- CN-U- 209 499 982
- CN-U- 209 499 982
- US-A1- 2014 243 966
- US-A1- 2019 209 303

## Description

The present application claims the priority of Chinese Patent No. 202010021982.8 filed on Jan. 9, 2020 with National Intellectual Property Administration, PRC, titled "STRUCTURALLY FITTED TRANSCATHETER AORTIC VALVE IMPLANTATION DEVICE".

### TECHNICAL FIELD

The present disclosure relates to the field of medical devices, and in particular, to a structurally fitted transcatheter aortic valve implantation device that is implantable via an approach through the aorta or through a transapical approach.

### BACKGROUND

About 300 thousand people worldwide are affected by cardiac valve diseases each year. Such diseases involve abnormal leaflet tissues, e.g., excess tissue growth, tissue degeneration or rupture, tissue hardening or calcifying, or abnormal tissue position throughout the cardiac cycle (i.e., annular dilation or ventricular reshaping), leading to dysfunction of valve, e.g., leakage or blood backflow (i.e., valve insufficiency) or resistance to forward blood flow (i.e., valve stenosis).

At present, existing transcatheter aortic valves rely on the inherent properties of the stent material and are simply secured at the position of the original aortic valve by friction. For example, Patent No. CN107890382A discloses a locatable and retrievable transcatheter aortic valve, wherein a first funnel opening structure of a valve stent is in contact with the left ventricular outflow tract and aortic annulus to serve as a support, and the valve stent has a locating rod structure configured for axial locating by securing the valve stent via friction between the lower part of the valve stent and surrounding tissue. However, due to the complexity of the pathological structures, the valve stent is positioned and secured by friction only, which may cause the valve to be pulled and pressed by the original structure after implantation and may cause valve migration, resulting in the risks of embolization, falling off or ejection, thereby causing failure of the valve implantation operation. Patent No. WO 2016/055262 A1 relates to an aortic valve replacement prosthesis which is radially compressible and re-expandable so as to be implantable via a catheter device, which may comprise a tubular body comprising a first longitudinal end portion, a second longitudinal end portion, and an intermediate portion connecting the first and second end portions with each other, wherein the tubular body has an inner circumferential surface and an outer circumferential surface, which extend at least substantially concentrically to the longitudinal axis, wherein the first and second longitudinal end portions and the intermediate portion of the tubular body are made of a wire-type structure including a plurality of interconnected first wire elements.

### SUMMARY

The invention ,which is defined by claim 1, therefore intends to provide a transcatheter aortic valve implantation device that is not solely fixed by friction. It forms structural matched with the blood vessels via a special design, accurately releases the valve at the aortic annulus, and avoids the adverse events caused by the existing fixation by friction alone, thus curing the aortic valve diseases. The invention is implemented by the following technical solutions:
The invention provides a structurally fitted transcatheter aortic valve implantation device comprising a valve stent, valve leaflets, an inner skirt and an outer skirt, wherein the valve stent is radially compressible and re-expandable so as to be implanted via a catheter device, and the valve stent comprises a tubular body having a circumference extending along a longitudinal axis, wherein the tubular body comprises an inner circumferential surface defining an inner cavity of the tubular body; an outer circumferential surface defining an outer surface of the tubular body, each of the inner circumferential surface and outer circumferential surface at least extending concentrically with the longitudinal axis; a first longitudinal end portion facing an ascending aorta side of a native aortic valve when the first longitudinal end portion is in an implanted state; a second longitudinal end portion facing a ventricular side of the native aortic valve when the second longitudinal end portion is in an implanted state; and an intermediate portion connecting the first and second longitudinal end portions, wherein a plurality of support arms are provided on the intermediate portion, the support arms being spaced from each other around the circumference of the tubular body and each of the support arms being integrally formed and directly connected on the tubular body and having an outer surface and; wherein the first and second longitudinal end portions and the intermediate portion of the tubular body are a grid-like structure; the valve leaflets are fixed on the intermediate portion in the inner cavity of the tubular body, the inner skirt is fixed at the second longitudinal end portion in the inner cavity of the tubular body and fixedly connected with the valve leaflets; and the outer skirt is fixed at the second longitudinal end portion in an outer cavity of the tubular body and fixedly connected with the inner skirt, wherein upon a complete expansion, the support arms are each in a letter "D"-shaped and can be fixed between a narrowest part (73) of an aorta in width close to the heart and a narrowest part of an aortic leaflet in width above an aortic annulus to allow the outer surfaces of the support arms to match with tissues surrounding the support arms; and when the device is implanted, the support arms are configured to expand horizontally at the aortic annulus, thereby moving the tubular body toward the ascending aorta side above the aortic annulus to be fixed at the narrowest part of the aorta in width close to the heart; characterized in that, each of the support arms further comprises a platform portion, an upper support arm and a lower support arm, the upper and lower support arms being formed tangentially with a smooth transition, and the platform portion being parallel to a direction of blood flow; each of the upper support arm, the lower support arm and the platform portion comprising a landing area in contact with the tissues surrounding the support arms, thereby allowing the outer surfaces of the support arms to match with the tissues surrounding the support arms;
and the landing areas are provided with two bending sections having the same length and a smaller width than the width of the landing areas to facilitate the each support arm to bend to form the letter "D" shape, the bending sections tapering from the landing areas.

According to the aortic valve implantation device disclosed herein, the three landing areas of the support arms have equal maximum widths.

According to the aortic valve implantation device disclosed herein, the support arms are spaced equidistantly or non-equidistantly around the circumference of the tubular body.

According to the aortic valve implantation device disclosed herein, the connections between the support arms and the tubular body taper from the landing areas to the bottoms.

According to the aortic valve implantation device disclosed herein, the tubular body and the support arms are machined by laser cutting.

According to the aortic valve implantation disclosed herein, the tubular body comprises a plurality of grid nodes, connected via grid elements, wherein in the intermediate portion the grid nodes are divided into a first node, a second node and a third node between the first node and the second node according to their respective positions along the longitudinal axis of the tubular body, two ends the each support arms are connected to the first node and the second node respectively, and a length of the grid elements between the first nodes and the second nodes to which the support arms are attached is greater than a length of the grid elements between the first nodes and the second nodes to which the support arms are not attached.

According to the aortic valve implantation device disclosed herein, a minimum width between the support arms and the grid elements that are adjacent to the support arms is configured to receive passage of one laser beam during the laser cutting, thereby maximizing the landing area of the support arms.

It should be noted that the dimensions and/or sizes used herein for describing the valve stent generally refer to a free expanded state of the valve stent, i.e., the expanded state other than any compressed circumstance. Thus, the dimensions and/or locations in a re-expanded implanted state may be different due to the compression provided by surrounding tissues.

### Beneficial Effects of Present Disclosure:

The present disclosure has advantages that structural matched of the aortic valve implantation device in the operation process is realized through the support arm structure located on the intermediate portion of the stent tubular body, thus reducing the risks of falling off, displacement or ejection in the process of implantation and increasing the success rate of valve implantation.

### BRIEF DESCRIPTION OF THE DRAWINGS

FIG. 1 shows a structural schematic view of an aortic valve implantation device according to the present disclosure;
FIG. 2 shows a schematic view of an embodiment of the aortic valve implantation device according to the present disclosure;
FIG. 3 shows a detailed view of a support arm of the aortic valve implantation device according to the present disclosure;
FIG. 4 shows another detailed view of the support arm of the aortic valve implantation device according to the present disclosure;
FIG. 5 shows a schematic view of the overall function formed by a plurality of support arms of the aortic valve implantation device according to the present disclosure;
FIG. 6 shows a schematic view of a delivery device for the transcatheter aortic valve implantation device according to the present disclosure; and
FIG. 7 shows a schematic view of the two-dimensional machining process of the intermediate portion and the support arms of the aortic valve implantation device according to the present disclosure.

### DETAILED DESCRIPTION

The present disclosure will be further illustrated with reference to the following specific examples. It should be understood that these examples are merely intended to illustrate the present disclosure rather than limit the protection scope of the present disclosure. In addition, it should be understood that various changes or modifications may be made by those skilled in the art after reading the teachings of present disclosure.

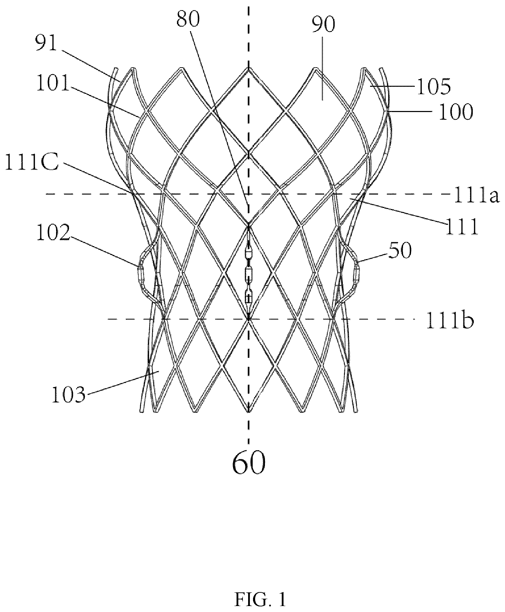

As shown in FIG. 1, a valve stent 100 comprises a tubular body 105, and the tubular body 105 consists of three portions, a first longitudinal end portion 101, an intermediate portion 102 and a second longitudinal end portion 103, which are made of a grid-like structure. The material of the tubular body 105 can be, for example, iron, nickel, aluminum, titanium, and/or alloys of these metals, and other elements. Reference numeral 111 denotes transparent artificial leaflet, each artificial leaflet 111 being connected to the inner cavity 90 of the tubular body 105. A region between two axial leaflets horizontal planes 111a and 111b that are longitudinally spaced apart from each other along the axis 60 of the tubular body 105 is a leaflet fixing region, wherein the axial leaflet horizontal plane 111a faces the first longitudinal end 101 and the axial leaflet horizontal plane 111b faces the second longitudinal end 103. The axial leaflet horizontal plane 111a spaces the first longitudinal end 101 apart from the intermediate portion 102. An artificial leaflet outflow supporting portion111c may be provided, which is connected to a corresponding grid element 80 to fix the artificial leaflet 111 on the tubular body 105. The axial leaflet horizontal plane 111b may be located around the second longitudinal end 103. The valve stent 100 may comprise an outer skirt and an inner skirt (not shown) made of animal pericardium or artificial material.

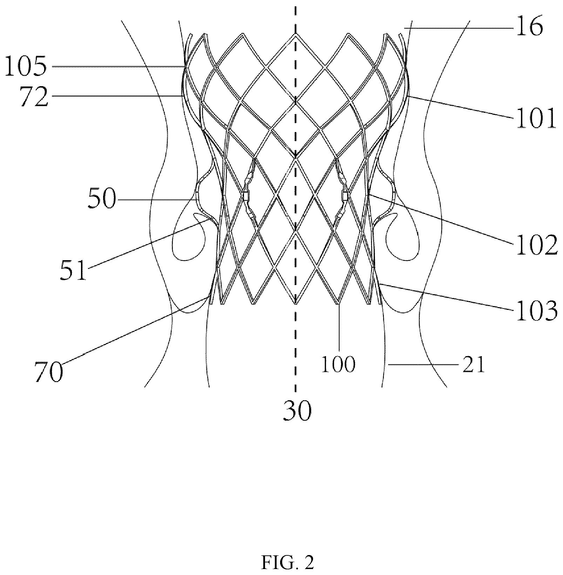

FIG. 2 shows an embodiment of the valve stent 100 for replacing a native aortic valve in a human or animal heart. That is, the valve stent 100 can be used as an artificial valve that allows blood to generally flow through the connecting channel in only one direction, in this embodiment, from the left ventricle 21 to the aorta 16, and may prevent leakage of blood in the direction from the aorta 16 to the left ventricle 21. The virtual longitudinal axis 30 is the longitudinal axis of the entire blood vessel.

When the valve stent 100 is implanted, i.e., in its implanted state, the first longitudinal end portion 101 faces the aorta 16 ipsilaterally to the ascending aorta 72, and the second longitudinal end portion 103 faces the left ventricle 21 ipsilaterally to the native aortic annulus 70.

The implanted valve stent 100 is movable in its expanded state in the direction towards the aortic side 16, with the support arms 50 protruding toward the outer surface 91 of the tubular body 105. Thereby the support arms 50 move longitudinally over the native aortic annulus 70 under its radial compression. As the support arms 50 have a specific profile and are free of hooks, barbs, kinks, etc., the support arms 50 do not become entangled with the body's native tissues or cause tissue damage when moving longitudinally.

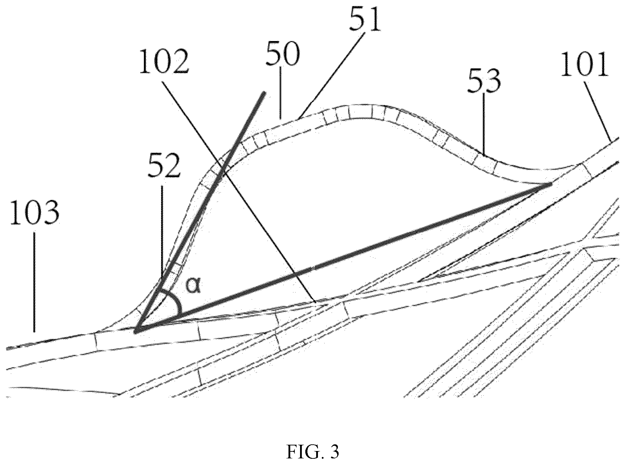

Referring to FIG. 3, the valve stent 100 may comprise a plurality of support arms 50, such as 3, 6, 9, 12 or more. The support arms 50 are integrally formed rather than being joined together by welding or other synthetic process. When the valve stent 100 expands, the support arms 50 may be circumferentially spaced from one another. The support arms 50 may be spaced from one another by equal circumferential distances, i.e., equidistantly distributed around the tubular body 105, or the support arms 50 may be spaced from one another by unequal circumferential distances. Each support arm 50 has an overall "D" shape and consists of three portions, a platform portion 51, an upper support arm 53 and a lower support arm 52. The "D" shaped design provides better integrity and anchoring function for structural matched in the transition region between the vessel and valve. The upper support arm 53, the lower support arm 52 and the platform portion 51 are centrosymmetrical. The upper support arm 53 is connected to the intermediate portion 102 towards the first longitudinal end portion 101, and the lower support arm 52 is connected to the intermediate portion 102 towards the second longitudinal end portion 103, the upper and lower support arms being formed tangentially with a smooth transition. The platform portion 51 is provided parallel to the direction of blood flow, rather than perpendicularly to the horizontal plane, and is capable of contacting the anatomical structure in the transition region of the vessel and valve in a parallel manner, minimizing the influence on blood flow.

As shown in FIG. 3, two angles are involved in the three-dimensional structure of the support arms, an upper angle and a lower angle. The upper angle and the lower angle are different in size. The lower angle α ranges from 45 degrees to 55 degrees, of which the selection is important for acquiring a D-shaped support arm with a maximum supporting force.

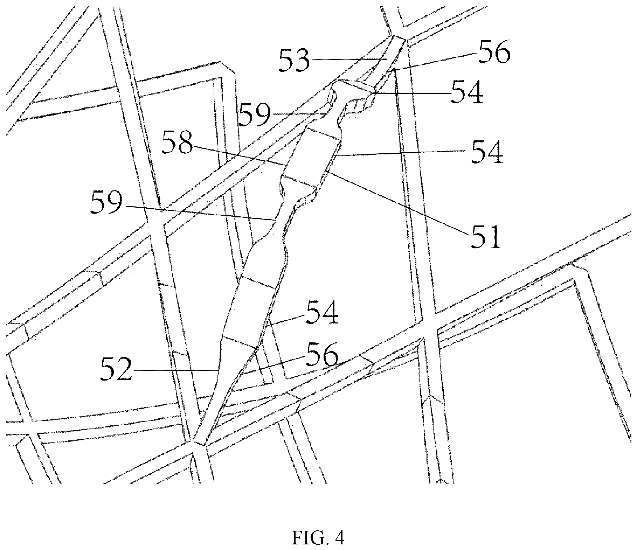

As shown in FIG. 4, the support arm 50 is further enlarged to better illustrate the formation of the structural match function. It can be seen that the upper support arm 53, lower support arm 52 and platform portion are provided with landing areas 54 for acquiring greater tension and/or compression when match occurs in the heart or vessel. Connections 56 between the support arm 50 and the implanted valve stent 100 taper from the landing areas 54 to the bottoms. Among the three landing areas 54 are provided two bending sections 59 of the same length, such that the support arms 50 are more easily bent to form a D-shaped configuration of the support arm.

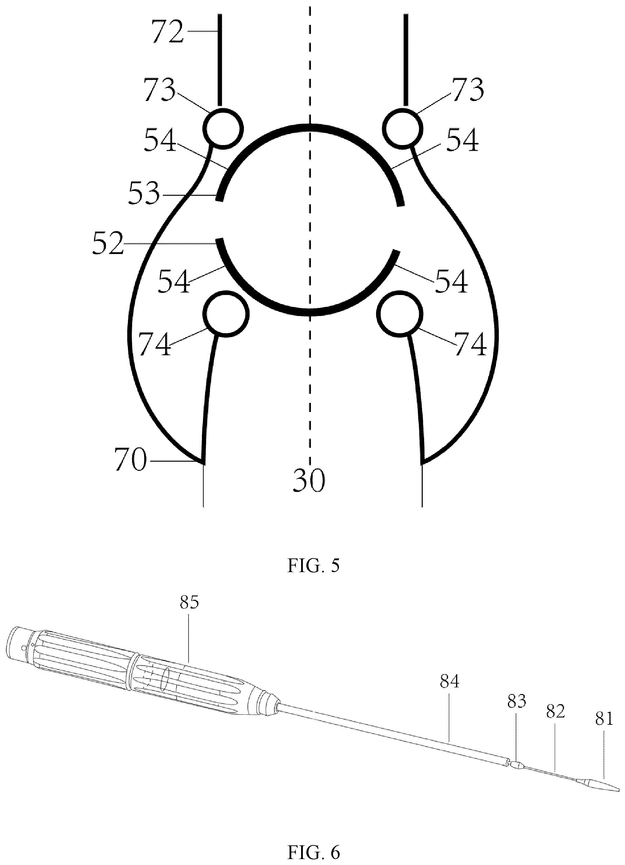

FIG. 5 schematically illustrates the function of the entirety formed by the plurality of support arms 50. The valve stent (not shown) forms an upper structural matched with the narrowest part 73 of the aorta close to the heart (e.g., the sinotubular junction, or other part of the ascending aorta) via the landing area 54 of the upper support arm 53. The valve stent (not shown) forms a lower structural matched with the narrowest part 74 above the aortic annulus (e.g., free edges of the aortic leaflet or the stenotic structures of the leaflet) via the landing area 54 of the lower support arm 52. This is clearly distinguished from existing prostheses implantation theories in the prior art relying solely on fixation by friction. The great risk of surgical failure due to fixation problems, such as displacement, falling off and ejection, is avoided by the structural matched.

Referring to FIG. 6, a schematic view of a delivery device 200 for the transcatheter aortic valve implantation device of the present disclosure is shown. The delivery device 80 of the embodiment of the present disclosure comprises: a delivery head 81, a prosthesis loading area 82, a loading site 83, a delivery catheter 84 and a rotatable grip handle 85. By the mating of the loading site 83 and the delivery head 81, the valve stent 100 is received in the prosthesis loading area 82. By slowly rotating the rotatable grip handle 85, the delivery catheter 84 will be advanced slowly (or retracted) to advance (or retract) the rotatable grip handle to complete the receiving (or release) of the valve stent from the prosthesis loading area 82.

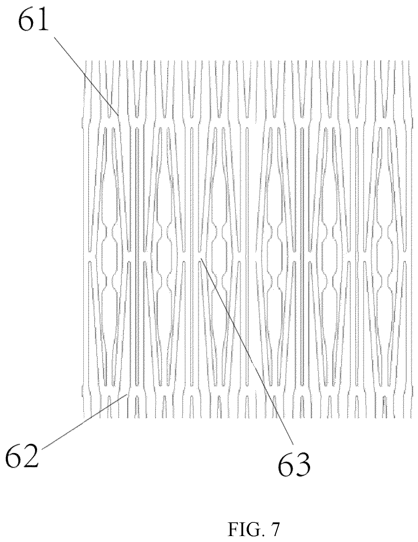

FIG. 7 is a schematic view of the two-dimensional machining process of the intermediate portion 102 and the support arms 50 of the tubular body, with the tubular body 105 and the support arms 50 being machined by laser cutting. The intermediate portion 102 of the tubular body 105 is provided with a plurality of grid nodes, connections among the grid nodes are grid elements, the grid nodes are divided into a first node 61, a second node 62 and a third node 63 according to axial positions, two ends of each of the support arms 50 are connected to the first node 61 and the second node 62 respectively, the third node 63 is located between the first node and the second node, and the grid elements between the first nodes and the second nodes to which the support arms are attached have a length greater than that of the grid elements between the first nodes and the second nodes to which the support arms are not attached.
and the grid elements between the first nodes 61 and the second nodes 62 to which the support arms are attached has a length greater than that of the grid elements between the first nodes 61 and the second nodes 62 to which the support arms are not attached. The process design allows an easier natural bending of the support arms 50 to a "D shaped configuration when the valve stent 100 expands.

Also, a minimum width between the support arms 50 and the adjacent grid elements can receive passage of only one laser beam during laser cutting, thereby maximizing the landing area 54 of the support arms 50. A larger landing area 54 ensures a larger contact area with the anatomical structures in the transition region of the vessel and valve, enabling a desired distribution of tension and facilitating the structural matched.

The support arms 50 is such designed that the maximum widths of the three landing areas 54 are equal, which allows an easier natural bending of the support arm to a "D" shaped configuration when the valve stent 100 expends.

In the grid structure of the tubular body, different widths of the structures are designed at different grid nodes according to different radial forces.

Examples of the present disclosure have been described above.

## Claims

1. A structurally fitted transcatheter aortic valve implantation device comprising a valve stent (100), valve leaflets, an inner skirt and an outer skirt, wherein
the valve stent (100) is radially compressible and re-expandable to facilitate a transcatheter implantation, and the valve stent comprises
a tubular body (105) having a circumference extending along a longitudinal axis, wherein the tubular body (105) comprises
an inner circumferential surface defining an inner cavity (90) of the tubular body (105);
an outer circumferential surface defining an outer surface (91) of the tubular body (105), each of the inner circumferential surface and outer circumferential surface at least extending concentrically with the longitudinal axis;
a first longitudinal end portion (101) facing an ascending aorta (72) side of a native aortic valve when the first longitudinal end portion (101) is in an implanted state;
a second longitudinal end portion (103) facing, a ventricular side of the native aortic valve when the second longitudinal end portion (103) is in an implanted state; and
an intermediate portion (102) connecting the first and second longitudinal end portions (101, 103), wherein a plurality of support arms (50) are provided on the intermediate portion (102), the support arms (50) being spaced from each other around the circumference of the tubular body (105) and each of the support arms (50) being integrally formed and directly connected on the tubular body (105) and having an outer surface and;
wherein the first and second longitudinal end portions (101, 103) and the intermediate portion (102) of the tubular body (105) are a grid-like structure;
the valve leaflets are fixed on the intermediate portion (102) in the inner cavity (90) of the tubular body (105),
the inner skirt is fixed at the second longitudinal end portion (103) in the inner cavity (90) of the tubular body (105) and fixedly connected with the valve leaflets; and
the outer skirt is fixed at the second longitudinal end portion (103) in an outer cavity of the tubular body (105) and fixedly connected with the inner skirt;
wherein upon a complete expansion, the support arms (50) are each in a letter "D" shape and can be fixed between a narrowest part (73) of an aorta (16) in width close to the heart and a narrowest part of an aortic leaflet in width above an aortic annulus (70) to allow the outer surfaces of the support arms (50) to match with tissues surrounding the support arms (50); and
when the device is implanted, the support arms (50) are configured to expand horizontally at the aortic annulus (70), thereby moving the tubular body (105) toward the ascending aorta (72) side above the aortic annulus (70) to be fixed at the narrowest part (73) of the aorta (16) in width close to the heart;
**characterized in that**,
each of the support arms (50) further comprises a platform portion (51), an upper support arm (53) and a lower support arm (52), the upper and lower support arms (50) being formed tangentially with a smooth transition, and the platform portion (51) being parallel to a direction of blood flow;
each of the upper support arm (53), the lower support arm (52) and the platform portion (51) comprising a landing area in contact with the tissues surrounding the support arms (50), thereby allowing the outer surfaces of the support arms (50) to match with the tissues surrounding the support arms (50);
and the landing areas (54) are provided with two bending sections (59) having the same length and a smaller width than the width of the landing areas (54) to facilitate the each support arm to bend to form the letter "D" shape,
the bending sections (59) tapering from the landing areas (54).

2. The structurally fitted transcatheter aortic valve implantation device according to claim 1, wherein the landing areas (54) of the support arms (50) have equal maximum widths.

3. The structurally fitted transcatheter aortic valve implantation device according to claim 2, wherein the support arms (50) are spaced equidistantly or non-equidistantly around the circumference of the tubular body (105).

4. The structurally fitted transcatheter aortic valve implantation device according to claim 3, wherein connections between the support arms (50) and the tubular body (105) taper from the landing areas (54).

5. The structurally fitted transcatheter aortic valve implantation device according to any one of claims 1-4, wherein the tubular body (105) and the support arms (50) are machined by laser cutting.

6. The structurally fitted transcatheter aortic valve implantation device according to claim 5, wherein the tubular body (105) comprises a plurality of grid nodes, connected via grid elements (80), wherein
in the intermediate portion (102), the grid nodes are divided into a first node (61), a second node (62) and a third node (63) between the first node (61) and the second node (62) according to their respective positions along the longitudinal axis of the tubular body (105), two ends of the each support arm (50) are connected to the first node (61) and the second node (62) respectively, and a length of the grid elements (80) between the first nodes (61) and the second nodes (62) to which the support arms (50) are attached is greater than a length of the grid elements (80) between the first nodes (61) and the second nodes (62) to which the support arms (50) are not attached.

7. The structurally fitted transcatheter aortic valve implantation device according to claim 6, wherein a minimum width between the support arms (50) and the grid elements (80) that are adjacent to the support arms (50) is configured to receive passage of one laser beam during the laser cutting, thereby maximizing the landing areas (54) of the support arms (50).

8. The structurally fitted transcatheter aortic valve implantation device according to claim 7, wherein the each support arm has a lower angle α ranging from 45 degrees to 55 degrees.

## Patentansprüche

1. Strukturell angepasste Transkatheter-Aortenklappenimplantationsvorrichtung, umfassend einen Klappenstent (100), Klappensegel, einen inneren Mantel und einen äußeren Mantel, wobei der Klappenstent (100) radial komprimierbar und wieder expandierbar ist, um eine Transkatheter-Implantation zu erleichtern, und der Klappenstent Folgendes umfasst:
einen rohrförmigen Körper (105) mit einem Umfang, der sich entlang einer Längsachse erstreckt, wobei der rohrförmige Körper (105) Folgendes umfasst:
eine innere Umfangsfläche, die einen inneren Hohlraum (90) des rohrförmigen Körpers (105) definiert;
eine äußere Umfangsfläche, die eine Außenfläche (91) des rohrförmigen Körpers (105) definiert, wobei sich jede der inneren Umfangsfläche und der äußeren Umfangsfläche mindestens konzentrisch zu der Längsachse erstreckt;
einen ersten Längsendabschnitt (101), der einer Seite einer aufsteigenden Aorta (72) einer nativen Aortenklappe zugewandt ist, wenn sich der erste Längsendabschnitt (101) in einem implantierten Zustand befindet;
einen zweiten Längsendabschnitt (103), der einer ventrikulären Seite der nativen Aortenklappe zugewandt ist, wenn sich der zweite Längsendabschnitt (103) in einem implantierten Zustand befindet; und
einen Zwischenabschnitt (102), der den ersten und zweiten Längsendabschnitt (101, 103) verbindet, wobei an dem Zwischenabschnitt (102) eine Vielzahl von Stützarmen (50) vorgesehen ist, die Stützarme (50) um den Umfang des rohrförmigen Körpers (105) voneinander beabstandet sind und jeder der Stützarme (50) einstückig ausgebildet und direkt mit dem rohrförmigen Körper (105) verbunden ist und eine Außenfläche aufweist; und
wobei der erste und der zweite Längsendabschnitt (101, 103) sowie der Zwischenabschnitt (102) des rohrförmigen Körpers (105) eine gitterartige Struktur aufweisen;
die Klappensegel am Zwischenabschnitt (102) im inneren Hohlraum (90) des rohrförmigen Körpers (105) fixiert sind,
der innere Mantel am zweiten Längsendabschnitt (103) im inneren Hohlraum (90) des rohrförmigen Körpers (105) fixiert und fest mit den Klappensegeln verbunden ist; und
der äußere Mantel am zweiten Längsendabschnitt (103) in einem äußeren Hohlraum des rohrförmigen Körpers (105) fixiert und fest mit dem inneren Mantel verbunden ist;
wobei die Stützarme (50) bei vollständiger Expansion jeweils die Form des Buchstabens "D" aufweisen und zwischen einem engsten Teil (73) einer Aorta (16) in der Breite nahe dem Herzen und einem engsten Teil eines Aortensegels in der Breite oberhalb eines Aortenrings (70) fixiert werden können, um zu ermöglichen, dass sich die Außenflächen der Stützarme (50) an das die Stützarme (50) umgebende Gewebe anpassen; und,
wenn die Vorrichtung implantiert ist, die Stützarme (50) so ausgelegt sind, dass sie am Aortenring (70) horizontal expandieren, wodurch der rohrförmige Körper (105) in Richtung der Seite der aufsteigenden Aorta (72) oberhalb des Aortenrings (70) bewegt wird, um am engsten Teil (73) der Aorta (16) in der Breite nahe dem Herzen fixiert zu werden,
**dadurch gekennzeichnet, dass**
jeder der Stützarme (50) ferner einen Plattformabschnitt (51), einen oberen Stützarm (53) und einen unteren Stützarm (52) umfasst, wobei der obere und der untere Stützarm (50) tangential mit einem sanften Übergang ausgebildet sind und der Plattformabschnitt (51) parallel zu einer Blutflussrichtung verläuft;
jeder von dem oberen Stützarm (53), dem unteren Stützarm (52) und dem Plattformabschnitt (51) eine Anlegefläche in Kontakt mit dem die Stützarme (50) umgebenden Gewebe umfasst, wodurch ermöglicht wird, dass sich die Außenflächen der Stützarme (50) an das die Stützarme umgebende Gewebe (50) anpassen;
und die Anlegeflächen (54) mit zwei Biegeabschnitten (59) versehen sind, die die gleiche Länge und eine geringere Breite als die Breite der Anlegeflächen (54) aufweisen, um zu erleichtern, dass sich jeder Stützarm biegt, um die Form des Buchstabens "D" zu bilden,
wobei sich die Biegeabschnitte (59) von den Anlegeflächen (54) verjüngen.

2. Strukturell angepasste Transkatheter-Aortenklappenimplantationsvorrichtung nach Anspruch 1, wobei die Anlegeflächen (54) der Stützarme (50) gleiche maximale Breiten aufweisen.

3. Strukturell angepasste Transkatheter-Aortenklappenimplantationsvorrichtung nach Anspruch 2, wobei die Stützarme (50) in gleichen oder ungleichen Abständen um den Umfang des rohrförmigen Körpers (105) angeordnet sind.

4. Strukturell angepasste Transkatheter-Aortenklappenimplantationsvorrichtung nach Anspruch 3, wobei sich Verbindungen zwischen den Stützarmen (50) und dem rohrförmigen Körper (105) von den Anlegeflächen (54) verjüngen.

5. Strukturell angepasste Transkatheter-Aortenklappenimplantationsvorrichtung nach einem der Ansprüche 1-4, wobei der rohrförmige Körper (105) und die Stützarme (50) durch Laserschneiden bearbeitet sind.

6. Strukturell angepasste Transkatheter-Aortenklappenimplantationsvorrichtung nach Anspruch 5, wobei der rohrförmige Körper (105) eine Vielzahl von Gitterknoten umfasst, die über Gitterelemente (80) verbunden sind, wobei
im Zwischenabschnitt (102) die Gitterknoten gemäß ihren jeweiligen Positionen entlang der Längsachse des rohrförmigen Körpers (105) in einen ersten Knoten (61), einen zweiten Knoten (62) und einen dritten Knoten (63) zwischen dem ersten Knoten (61) und dem zweiten Knoten (62) unterteilt sind, zwei Enden jedes der Stützarme (50) mit dem ersten Knoten (61) bzw. dem zweiten Knoten (62) verbunden sind und eine Länge der Gitterelemente (80) zwischen den ersten Knoten (61) und den zweiten Knoten (62), an denen die Stützarme (50) befestigt sind, größer ist als eine Länge der Gitterelemente (80) zwischen den ersten Knoten (61) und den zweiten Knoten (62), an denen die Stützarme (50) nicht befestigt sind.

7. Strukturell angepasste Transkatheter-Aortenklappenimplantationsvorrichtung nach Anspruch 6, wobei eine Mindestbreite zwischen den Stützarmen (50) und den Gitterelementen (80), die benachbart zu den Stützarmen (50) sind, so ausgelegt ist, dass sie den Durchtritt eines Laserstrahls während des Laserschneidens aufnimmt, wodurch die Anlegeflächen (54) der Stützarme (50) maximiert werden.

8. Strukturell angepasste Transkatheter-Aortenklappenimplantationsvorrichtung nach Anspruch 7, wobei jeder der Stützarme einen flacheren Winkel α im Bereich von 45 Grad bis 55 Grad aufweist.

## Revendications

1. Dispositif d'implantation de valve aortique transcathéter ajusté structurellement comprenant une endoprothèse valvulaire (100), des feuillets valvulaires, une jupe intérieure et une jupe extérieure, dans lequel
l'endoprothèse valvulaire (100) est compressible radialement et ré-expansible pour faciliter une implantation transcathéter, et l'endoprothèse valvulaire comprend
un corps tubulaire (105) ayant une circonférence s'étendant le long d'un axe longitudinal, dans lequel le corps tubulaire (105) comprend
une surface circonférentielle intérieure définissant une cavité intérieure (90) du corps tubulaire (105) ;
une surface circonférentielle extérieure définissant une surface extérieure (91) du corps tubulaire (105), chacune de la surface circonférentielle intérieure et de la surface circonférentielle extérieure s'étendant au moins concentriquement avec l'axe longitudinal ;
une première portion d'extrémité longitudinale (101) faisant face à un côté d'aorte ascendante (72) d'une valve aortique native lorsque la première portion d'extrémité longitudinale (101) est dans un état implanté ;
une deuxième portion d'extrémité longitudinale (103) faisant face à un côté ventriculaire de la valve aortique native lorsque la deuxième portion d'extrémité longitudinale (103) est dans un état implanté ; et
une portion intermédiaire (102) raccordant les première et deuxième portions d'extrémité longitudinales (101, 103), dans lequel une pluralité de bras de support (50) sont prévus sur la portion intermédiaire (102), les bras de support (50) étant espacés les uns des autres autour de la circonférence du corps tubulaire (105) et chacun des bras de support (50) étant formé de manière solidaire et directement raccordé sur le corps tubulaire (105) et ayant une surface extérieure et ;
dans lequel les première et deuxième portions d'extrémité longitudinales (101, 103) et la portion intermédiaire (102) du corps tubulaire (105) sont une structure en forme de grille ;
les feuillets valvulaires sont fixés sur la portion intermédiaire (102) dans la cavité intérieure (90) du corps tubulaire (105),
la jupe intérieure est fixée à la deuxième portion d'extrémité longitudinale (103) dans la cavité intérieure (90) du corps tubulaire (105) et raccordée de manière fixe aux feuillets valvulaires ; et
la jupe extérieure est fixée à la deuxième portion d'extrémité longitudinale (103) dans une cavité extérieure du corps tubulaire (105) et raccordée de manière fixe à la jupe intérieure ;
dans lequel, une fois en expansion complète, les bras de support (50) ont chacun la forme d'une lettre « D » et peuvent être fixés entre une partie la plus étroite (73) d'une aorte (16) en largeur près du cœur et une portion la plus étroite d'une valvule de l'aorte en largeur au-dessus de l'anneau aortique (70) pour permettre aux surfaces extérieures des bras de support (50) de concorder avec les tissus entourant les bras de support (50) ; et
lorsque le dispositif est implanté, les bras de support (50) sont configurés pour s'étendre horizontalement au niveau de l'anneau aortique (70), déplaçant ainsi le corps tubulaire (105) vers l'aorte ascendante (72) du côté au-dessus de l'anneau aortique (70) pour être fixé à la partie la plus étroite (73) de l'aorte (16) en largeur près du cœur ;
**caractérisé en ce que**,
chacun des bras de support (50) comprend en outre une portion plateforme (51), un bras de support supérieur (53) et un bras de support inférieur (52), les bras de support (50) supérieur et inférieur étant formés tangentiellement avec une transition douce, et la portion plateforme (51) étant parallèle à une direction de l'écoulement sanguin ;
chacun du bras de support supérieur (53), du bras de support inférieur (52) et de la portion plateforme (51) comprenant une zone d'appui en contact avec les tissus entourant les bras de support (50), permettant ainsi aux surfaces extérieures des bras de support (50) de concorder avec les tissus environnants des bras de support (50) ;
et les zones d'appui(54) sont dotées de deux sections de flexion (59) de même longueur et d'une largeur plus petite que celle des zones d'appui (54) afin de faciliter la flexion de chaque bras de support pour former la forme de la lettre « D »,
les sections de flexion (59) s'effilant à partir des zones d'appui (54).

2. Dispositif d'implantation de valve aortique transcathéter ajusté structurellement selon la revendication 1, dans lequel les zones d'appui (54) des bras de support (50) ont des largeurs maximales égales.

3. Dispositif d'implantation de valve aortique transcathéter ajusté structurellement selon la revendication 2, dans lequel les bras de support (50) sont espacés de manière équidistante ou non équidistante autour de la circonférence du corps tubulaire (105).

4. Dispositif d'implantation de valve aortique transcathéter ajusté structurellement selon la revendication 3, dans lequel les raccords entre les bras de support (50) et le corps tubulaire (105) s'effilent à partir des zones d'appui (54).

5. Dispositif d'implantation de valve aortique transcathéter ajusté structurellement selon l'une quelconque des revendications 1 à 4, dans lequel le corps tubulaire (105) et les bras de support (50) sont usinés par découpe laser.

6. Dispositif d'implantation de valve aortique transcathéter ajusté structurellement selon la revendication 5, dans lequel le corps tubulaire (105) comprend une pluralité de nœuds de grille, raccordés par l'intermédiaire d'éléments de grille (80), dans lequel
dans la portion intermédiaire (102), les nœuds de grille sont divisés en un premier nœud (61), un deuxième nœud (62) et un troisième nœud (63) entre le premier nœud (61) et le deuxième nœud (62) selon leurs positions respectives le long de l'axe longitudinal du corps tubulaire (105), deux extrémités de chaque bras de support (50) sont raccordées respectivement au premier nœud (61) et au deuxième nœud (62), et une longueur des éléments de grille (80) entre les premiers nœuds (61) et les deuxièmes nœuds (62) auxquels les bras de support (50) sont attachés est supérieure à une longueur des éléments de grille (80) entre les premiers nœuds (61) et les deuxièmes nœuds (62) auxquels les bras de support (50) ne sont pas attachés.

7. Dispositif d'implantation de valve aortique transcathéter ajusté structurellement selon la revendication 6, dans lequel une largeur minimale entre les bras de support (50) et les éléments de grille (80) qui sont adjacents aux bras de support (50) est configurée pour recevoir le passage d'un faisceau laser pendant la découpe laser, maximisant ainsi les zones d'appui (54) des bras de support (50).

8. Dispositif d'implantation de valve aortique transcathéter ajusté structurellement selon la revendication 7, dans lequel chaque bras de support a un angle inférieur α allant de 45 degrés à 55 degrés.
